# FASCICULE DE BREVET EUROPEEN

(11) **EP 2 621 896 B1**
(45) Date de publication et mention de la délivrance du brevet: **12.12.2018**
(21) Numéro de dépôt: 11779768.8
(22) Date de dépôt: 30.09.2011
(51) Int. Cl.: C07C 319/28, C07C 321/14

(54) **PROCÉDÉ DE PRÉPARATION DE DISULFURE DE DIMÉTHYLE**
VERFAHREN ZUR HERSTELLUNG VON DIMETHYLDISULFIDE
PROCESS FOR PREPARING DIMETHYL DISULFIDE

(30) Priorité: 30.09.2010 FR 1057904
(43) Date de publication de la demande: 07.08.2013
(73) Titulaire: Arkema France, 92700 Colombes (FR)
(72) Inventeur: FREMY, Georges, F-64390 Sauveterre De Bearn (FR); RAYMOND, Jean-Michel, F-40300 Cauneille (FR)
(86) Numéro de dépôt international: PCT/FR2011/052285
(87) Numéro de publication internationale: WO 2012/042184

(56) Documents cités:
- EP-A1- 0 976 726

## Description

La présente invention concerne le domaine des di-sulfures et poly-sulfures organiques (dits « sulfures organiques » dans la suite) et plus particulièrement celui du disulfure de diméthyle (ou diméthyldisulfure ou DMDS), et notamment son procédé de préparation.

Les sulfures organiques sont largement utilisés dans de très nombreux domaines de l'industrie chimique et notamment pétrochimique. En particulier, le disulfure de diméthyle est utilisé comme agent de sulfuration de catalyseurs d'hydrotraitement de charges pétrolières, en tant qu'additif de charge pour le vapocraquage, pour ne citer que quelques une des utilisations possibles de ce composé.

Par rapport à d'autres produits utilisés dans ces applications, comme par exemple les di-*tertio*-alkylpolysulfures commerciaux, les sulfures organiques, et en particulier le DMDS, présentent de nombreux avantages, notamment une teneur en soufre élevée (68 %) et des produits de dégradation non cokants (CH₄, H₂S dans le cas du DMDS). De plus, dans ces applications, le DMDS conduit à des performances généralement supérieures aux autres produits commerciaux et habituellement utilisés, par exemple les di-*tertio*-alkylpolysulfures.

Parmi les méthodes connues pour les synthèses des sulfures organiques, une méthode particulièrement efficace et économique est l'oxydation des alkylmercaptans par le soufre, par exemple, pour la synthèse de DMDS, l'oxydation par le soufre du méthylmercaptan selon la réaction suivante :

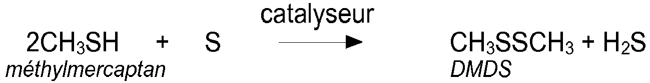

Cette oxydation des alkylmercaptans par le soufre, catalysée par des agents basiques organiques ou inorganiques, homogènes ou hétérogènes, en discontinu ou en continu, s'accompagne d'un dégagement d'hydrogène sulfuré ainsi que de dialkylpolysulfures (par exemple polysulfures de diméthyle CH₃SₓCH₃ dans le cas de la synthèse de DMDS) de rang en soufre x supérieur à 2.

Pour fabriquer le DMDS selon ce procédé d'oxydation par le soufre avec des rendements élevés et une production limitée en DMPS (diméthylpolysulfures de rang supérieur à 2), le brevet EP 0 446 109 décrit un procédé de préparation comprenant deux zones de réaction interrompues par une zone de dégazage intermédiaire et suivies d'une zone de distillation, pour éliminer les sous-produits indésirables.

Bien que performant en termes de rendement et de sélectivité en DMDS, il s'avère que ce procédé conduit à un produit fini comprenant une quantité non négligeable d'hydrodisulfure de méthyle (CH₃SSH). Cette quantité varie généralement entre environ 200 ppm et environ 800 ppm, en particulier lorsque des productivités élevées en DMDS sont recherchées.

La résultante de cette impureté, qui peut être considérée comme un intermédiaire réactionnel, est une lente décomposition dans le temps en méthylmercaptan et en diméthyltrisulfure, par réaction avec le diméthyldisulfure, selon la réaction suivante :

CH₃SSH + CH₃SSCH₃ → CH₃SH + CH₃SSSCH₃

L'instabilité de l'hydrodisulfure de méthyle (CH₃SSH, N° CAS : 6251-26-9) est par ailleurs mentionnée dans la littérature par H. Bohme et G. Zinner, dans « Justus Liebigs Annalen der Chemie », 585, (1954), 142-9, où l'hydrodisulfure de méthyle CH₃SSH se décompose à température ambiante, en réagissant sur lui-même, pour donner du diméthyltrisulfure et de l'H₂S selon la réaction :

2CH₃SSH → H₂S + CH₃SSSCH₃

Dans le cas de la synthèse du DMDS par oxydation au soufre, les très faibles concentrations en CH₃SSH ne permettent pas à ce produit de réagir sur lui-même, et la réaction avec le produit majoritairement présent dans le milieu, le DMDS, est extrêmement favorisée.

L'augmentation de la teneur en méthylmercaptan dans le DMDS rend la synthèse de DMDS décrite dans la demande EP 0 976 726 peu rentable, en raison des impuretés volatiles du DMDS (traces de méthylmercaptan et traces de sulfure de diméthyle (DMS)) qui sont éliminées dans une étape supplémentaire de distillation.

En effet, ses impuretés confèrent au DMDS une odeur très désagréable et agressive, qui est considérée comme une gêne importante lors de la manipulation de ce produit par les utilisateurs.

Cependant, le DMDS obtenu, débarrassé des impuretés volatiles, contient encore des traces de CH₃SSH. En outre, la simple élimination par distillation des impuretés conduit à des pertes importantes de rendement en DMDS. En effet, le CH₃SSH est beaucoup moins volatil que le méthylmercaptan et que le DMS, et présente un point d'ébullition de 108°C très proche de celui du DMDS (point d'ébullition compris entre 107°C et 110°C à pression ambiante). Ces produits (DMDS et CH₃SSH) sont donc très difficilement séparables.

La présente invention a donc pour principal objectif de proposer un procédé permettant de réduire, voire d'éliminer en très grande partie jusqu'à totalement, l'impureté CH₃SSH dans la synthèse du DMDS.

La présente invention a également pour objectif un procédé d'élimination de l'impureté sans perte significative de rendement en DMDS.

Il a maintenant été trouvé de manière surprenante que la vitesse de décomposition de CH₃SSH en présence de DMDS correspondant peut être grandement augmentée par passage sur un catalyseur basique d'un brut réactionnel comprenant majoritairement ledit DMDS.

Dans la suite de la présente description on entend par brut réactionnel comprenant majoritairement du DMDS un milieu réactionnel dans lequel a été réalisée, par catalyse basique, l'oxydation par le soufre du méthylmercaptan.

Le brut réactionnel est un brut réactionnel duquel l'hydrogène sulfuré a été séparé (stripping) et duquel le méthylmercaptan n'ayant pas réagi a également été séparé ( par distillation).

Le brut réactionnel est un brut réactionnel duquel non seulement l'hydrogène sulfuré et le méthylmercaptan résiduel, mais aussi les polysulfures (« lourds »), ainsi qu'éventuellement les traces d'alkylmercaptan et de sulfure d'alkyle, sont séparés, par exemple par distillation.

Ainsi, et selon un premier aspect, la présente invention concerne un procédé de préparation de disulfure de diméthyle, ledit procédé comprenant au moins les étapes suivantes :
a) oxydation par le soufre de méthylmercaptan, par catalyse basique, pour obtenir un premier brut réactionnel contenant majoritairement le disulfure de diméthyle correspondant ;
b) stripping du H₂S formé ;
c) distillation du méthylmercaptan résiduel ;
d) séparation des produits lourds (polysulfures) ;
e) étêtage final permettant d'éliminer les dernières traces des composés volatils ;
f) mise en contact du flux en sortie de l'étape d), ou en sortie de l'étape e) avec un catalyseur basique permettant l'élimination de l'hydrodisulfure de méthyle par conversion dudit hydrodisulfure de méthyle en sulfure ou polysulfures de diméthyle ;
g) récupération du disulfure de diméthyle à faible teneur en hydrodisulfure de méthyle.

Par faible teneur en hydrodisulfure de méthyle, on entend une teneur généralement inférieure à 50 ppm en poids, de préférence inférieure à 10 ppm en poids, de préférence encore inférieure à 5 ppm en poids.

L'étape a) du procédé décrit ci-dessus d'oxydation de méthylmercaptan par le soufre est conduite de manière classique et connue de l'homme du métier. Cette étape est par exemple décrite dans la demande de brevet EP 0 976 726. Par exemple l'étape a) peut être conduite à chaud et sous pression, par exemple entre 20°C et 100°C, sous 2 à 15 bars, typiquement par exemple à environ 70°C, sous environ 6 bars dans le cas de l'oxydation par le soufre du méthylmercaptan.

Les étapes b), c), d) et e) sont comprises comme des étapes de purification, c'est-à-dire à l'élimination au moins partielle et idéalement totale, des sous-produits non désirables présents dans le brut réactionnel obtenu à l'issue de l'étape a). Plus précisément chacune des étapes b), c), d) et e) correspond à l'élimination de H₂S, de méthylmercaptan (réactif de départ), des produits lourds, et des traces des composés volatils, respectivement.

Chacune de ces étapes dites de « purification » d) et e) peuvent être conduites selon toutes méthodes connues de l'homme du métier, telles que strippping, distillation, décantation, et autres.

L'étape f) d'élimination de l'hydrodisulfure de méthyle, en sulfure ou polysulfures de diméthyle correspond quant à elle à une réaction chimique ultérieure et séparée conduite en présence d'un catalyseur basique, et qui est effectuée après l'étape d).

Ainsi, et selon un mode de réalisation tout particulièrement préféré, l'étape f) de conversion par catalyse basique est effectuée juste avant l'étape e) d'étêtage final.

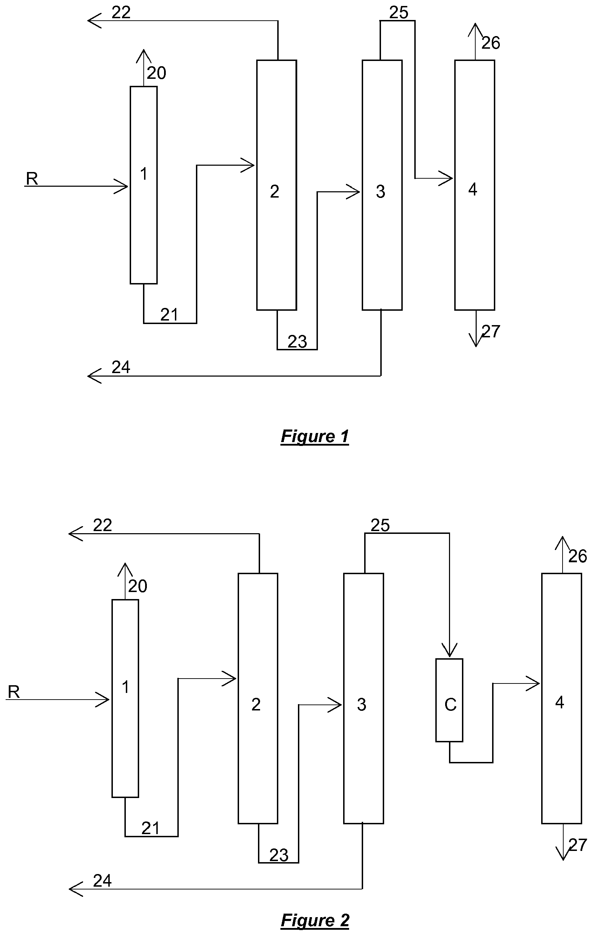

Pour la synthèse de disulfure de diméthyle, l'étape f) de catalyse basique est avantageusement effectuée après l'étape d) de séparation des produits lourds, c'est-à-dire après la colonne 6 de distillation des produits lourds et avant la dernière colonne 7 de distillation décrite à la Figure 2 de la demande de brevet EP 0 976 726.

.Le procédé selon l'invention est ainsi tout particulièrement adapté à la synthèse de disulfure de diméthyle, sans perte de rendement, avec élimination de l'impureté hydrodisulfure de méthyle. L'élimination de l'hydrodisulfure est réalisée au moyen d'au moins un catalyseur basique.

Le catalyseur basique peut être de tout type connu de l'homme du métier. Ledit catalyseur basique est de préférence hétérogène par rapport au milieu réactionnel, de manière à faciliter sa séparation ultérieure. Ainsi, le catalyseur basique peut par exemple être choisi parmi les résines échangeuses d'anions, telle que l'Amberlyst® A21 de Rohm & Haas, les catalyseurs basiques sous forme amine libre, les alumines dopée à l'oxyde de sodium et/ou à l'oxyde de potassium, l'oxyde de magnésium (MgO), et autres. De préférence le catalyseur basique est une résine échangeuse d'anions.

La réaction de catalyse basique du brut réactionnel est conduite dans une étape séparée et ultérieure à la synthèse par oxydation au soufre. Autrement dit, le brut réactionnel est engagé, avantageusement après avoir été soumis à une ou plusieurs purifications telles que celles exposées dans la suite, à une nouvelle étape consistant en une catalyse basique qui permet d'éliminer l'impureté hydrodisulfure de méthyle, sans perte significative de rendement en disulfure de diméthyle.

Les inventeurs ont en effet découvert de manière surprenante que l'on peut fortement accélérer la vitesse de décomposition de l'hydrodisulfure de méthyle, en présence du disulfure de diméthyle, dans une réaction ultérieure et séparée de catalyse basique.

Selon un mode de réalisation préféré, le catalyseur basique utilisé pour réduire la teneur en hydrodisulfure de méthyle du brut réactionnel comprenant majoritairement le disulfure de méthyle, est du même type, voire est identique au catalyseur basique utilisé pour la réaction d'oxydation du méthylmercaptan par le soufre pour conduire audit brut réactionnel comprenant majoritairement du disulfure de diméthyle.

Un autre avantage lié au procédé selon la présente invention est qu'il permet d'augmenter la production industrielle du disulfure de diméthyle. En effet, dans les procédés classiquement utilisés aujourd'hui, par exemple comme décrit dans la demande de brevet EP 0 976 726, l'opérateur cherche à minimiser la formation d'hydrodisulfure organique responsable, par auto-dégradation comme indiqué plus haut, des mauvaises odeurs desdits disulfures de diméthyle. Un moyen pour limiter la formation d'hydrodisulfure consiste à ralentir la réaction d'oxydation du méthylmercaptan par le soufre en limitant la vitesse d'introduction dudit alkylmercaptan dans la réaction d'oxydation.

Grâce au procédé de la présente invention, qui consiste à ajouter au procédé conventionnel une étape de catalyse basique du brut réactionnel permettant la conversion de l'hydrodisulfure de méthyle, il est maintenant possible d'augmenter la vitesse d'introduction du méthylmercaptan de départ d'environ 30% à 100%. Ceci permet d'augmenter considérablement la productivité globale de la synthèse industrielle de disulfure de diméthyle.

Par exemple, dans le cas de la synthèse de disulfure de diméthyle, le débit initial de méthylmercaptan peut être augmenté de environ 950 g/h (dans le procédé classique, sans catalyse basique pour la conversion de l'hydrodisulfure de méthyle) jusqu'à environ 1500 g/h avec l'étape supplémentaire de catalyse basique du brut réactionnel selon la présente invention.

Autrement dit, on a observé, par rapport à une synthèse sans réacteur catalytique de « finissage », une augmentation de la productivité par m³ de catalyseur (de la réaction principale d'oxydation par le soufre) d'environ 2000 kg/h/m³ de catalyseur à environ 3000 kg/h/m³ de catalyseur (de la réaction principale d'oxydation par le soufre).

La Figure 1 annexée est un schéma présentant une installation industrielle de purification de brut réactionnel de synthèse de disulfure de diméthyle, comme décrit dans la demande de brevet EP 0 976 726.

Une colonne de dégazage **1** sert à éliminer complètement, via la conduite **20**, l'H₂S dissous dans le brut réactionnel sortant du réacteur, via la conduite **R**. Une colonne de distillation **2** permet de séparer la majeure partie du méthylmercaptan en excès en vue de son recyclage par la conduite **22** dans la réaction d'oxydation.

À la sortie de la colonne **2**, le mélange est amené par la conduite **23** dans la deuxième colonne de distillation **3** où les polysulfures de diméthyle sont éliminés en fond de colonne par la conduite 24 pour être éventuellement recyclés dans la réaction d'oxydation.

Le disulfure de diméthyle, recueilli en tête de la colonne **3** par la conduite **25** est introduit dans une troisième colonne de distillation **4** où les impuretés volatiles, telles que méthylmercaptan et sulfure de diméthyle sont éliminés en tête de colonne par la conduite **26**. Le disulfure de diméthyle est recueilli en fond de colonne par la conduite **27**.

La Figure 2 annexée est un schéma similaire à celui présenté à la Figure 1, avec en outre le réacteur C de catalyse basique selon la présente invention. Dans la Figure 2, qui représente un mode de réalisation préféré de la présente invention, le réacteur C permettant la catalyse basique est placé entre la colonne 3 et la colonne 4, c'est-à-dire après l'étape d) de séparation des produits lourds et avant l'étape e) d'étêtage final.

La présente invention est maintenant illustrée par les exemples suivants, qui ne présentent aucun caractère limitatif pour l'invention telle que revendiquée dans les revendications annexées.

Dans ces exemples, la vitesse de passage sur le catalyseur basique de conversion de l'hydrodisulfure de méthyle, que l'on appellera vitesse volumique horaire (WH) est exprimée en h⁻¹ et est égale au rapport du débit volumique horaire de réactif sur le volume dudit catalyseur basique de conversion.

### EXEMPLE 1 : Synthèse du DMDS selon EP 0 976 726 avec conditions de productivités plus élevées, et sans catalyse basique de conversion (exemple comparatif)

### a) Appareillage :

La Figure 1 annexée est un schéma de l'installation utilisée, dans laquelle un brut réactionnel issu de la réaction de l'oxydation de méthylmercaptan par le soufre (comme décrit dans la demande de brevet EP 0 976 726).

Une colonne de dégazage 1 sert à éliminer complètement, via la conduite 20, l'H₂S dissous dans le brut réactionnel sortant du réacteur, via la conduite R. Une colonne de distillation 2 permet de séparer la majeure partie du méthylmercaptan en excès en vue de son recyclage par la conduite 22 dans la réaction d'oxydation.

La colonne 3 permet de séparer les polysulfures de diméthyle résiduels (DMPS) en vue de leur recyclage dans le réacteur d'oxydation par la conduite 24.

### b) Mode opératoire :

Le méthylmercaptan (MM) est introduit à l'état liquide sous pression avec un débit de 1440 g/h dans le réacteur. Le soufre liquide (S) est introduit avec un débit de 240 g/h dans un premier réacteur (ratio molaire MM/S = 4).

Le réacteur d'oxydation (volume réactionnel de 300 mL) contient 20 g de résine Amberlyst® A21 sèche. La pression opératoire est maintenue à 5,5 bars relatifs et la température à 40°C. Le mélange réactionnel en sortie de ce premier réacteur est alors envoyé dans un dégazeur pour être traité.

Après traitement, le mélange débarrassé de l'H₂S est envoyé dans un second réacteur qui contient une charge de 94 g de résine Amberlyst® A21 sèche. La pression dans ce second réacteur est de 5,5 bars relatifs et la température de 50°C. À la sortie du réacteur, le mélange est alors introduit dans un dégazeur pour l'élimination de l'H₂S.

À la sortie de la colonne de dégazage 1, le mélange est introduit dans la première colonne de distillation 2 par la conduite 21 pour éliminer la quasi totalité du méthylmercaptan en excès. Ce méthylmercaptan peut être recyclé par la conduite 22 à l'introduction des réactifs dans la réaction d'oxydation. À la sortie de la colonne 2, le mélange est amené par la conduite 23 dans la deuxième colonne de distillation 3 où les DMPS sont éliminés en fond de colonne par la conduite 24 pour être éventuellement recyclés dans la réaction d'oxydation.

Le DMDS, recueilli en tête de la colonne 3 par la conduite 25 est introduit dans une troisième colonne de distillation 4 où les impuretés volatiles telles que le méthylmercaptan et le sulfure de diméthyle sont éliminés en tête de colonne par la conduite 26. Le DMDS recueilli en fond de colonne par la conduite 27 a la composition pondérale suivante (toutes les valeurs sont exprimées en poids) :

| | | |
|---|---|---|
| - DMDS : | 99,9 | % |
| - DMTS : | 334 | ppm |
| - CH₃SSH : | 377 | ppm |
| - MM : | 32 | ppm |
| - DMS : | < 2 | ppm (limite de détection) |

Cet essai montre la présence de CH₃SSH dans le produit fini lorsque l'on réalise la synthèse du DMDS dans des conditions de productivité plus élevée que dans la demande de brevet EP 0 976 726 (débits des réactifs et température du réacteur d'oxydation plus élevés).

### EXEMPLE 2 : conforme à la présente invention

Soit le brut de DMDS ci-dessus de l'exemple 1 contenant 377 ppm en poids de CH₃SSH. Ces 377 ppm peuvent redonner dans le temps 226 ppm de CH₃SH dans le produit fini.

Ce DMDS est envoyé sur un lit de résine basique Amberlyst® A21 de Rohm & Haas qui a été préalablement lavée au méthanol et séchée pour éliminer l'eau contenue dans cette résine (5 g de résine sèche, soit 14,3 mL) à raison de 180 g/h (171 mL/h ce qui conduit à une VVH de 12 h⁻¹) pendant 2 heures et à une température de 40°C. Le CH₃SSH n'est plus détectable par chromatographie gazeuse (limite de détection environ 2 ppm en poids) en sortie du réacteur tubulaire contenant la résine.

### EXEMPLE 3 :

Soit un autre brut de DMDS contenant cette fois 428 ppm de CH₃SSH (ces 428 ppm peuvent redonner dans le temps 257 ppm de CH₃SH dans le produit fini).

Ce DMDS est envoyé sur un lit de résine basique Amberlyst® A21 de Rohm & Haas préalablement séchée (5 g, soit 14,3 mL) à raison de 530 g/h (505 mL/h, soit une VVH de 35 h⁻¹) pendant 2 heures et à une température de 40°C. Le CH₃SSH n'est plus détectable par chromatographie gazeuse (limite de détection environ 2 ppm en poids) en sortie du réacteur tubulaire contenant la résine.

### EXEMPLE 4

Soit un brut de DMDS contenant 219 ppm de CH₃SSH (ces 219 ppm peuvent redonner dans le temps 131 ppm de CH₃SH dans le produit fini).

Ce DMDS est envoyé sur un lit de résine basique Amberlyst® A21 de Rohm & Haas préalablement séchée (5 g, soit 14,3 mL), à raison de 1000 g/h (952 mL/h soit une VVH de 67 h⁻¹) pendant 2 heures et ensuite à 2000 g/h (1904 mL/h soit une VVH de 133 h⁻¹) pendant 2 heures également et à une température de 40°C.

À ces 2 débits, le CH₃SSH n'est plus détectable par chromatographie gazeuse (limite de détection d'environ 2 ppm en poids) en sortie du réacteur tubulaire contenant la résine.

### EXEMPLE 5

Soit un brut de DMDS contenant 239 ppm de CH₃SSH (ces 239 ppm peuvent redonner dans le temps 140 ppm de CH₃SH dans le produit fini).

Ce DMDS est envoyé sur un lit de résine basique Amberlyst® A21 de Rohm & Haas préalablement séchée (5 g, soit 14,3 mL) à raison de 2000 g/h (1904 mL/h soit une VVH de 133 h⁻¹) pendant 2 heures et à une température de 20°C.

Le CH₃SSH n'est plus détectable par chromatographie gazeuse (limite de détection : environ 2 ppm en poids) en sortie du réacteur tubulaire contenant la résine.

Le DMDS utilisé dans cet exemple qui contenait initialement 441 ppm de trisulfure de diméthyle (DMTS), en contient après passage sur la résine 809 ppm, soit une augmentation de 368 ppm. Cette valeur de 368 ppm est très proche du maximum théorique qui est de 376 ppm (valeur que l'on obtient en considérant que chaque mole de CH₃SSH a donné une mole de DMTS).

À ces 2 débits, le CH₃SSH n'est plus détectable par chromatographie gazeuse (limite de détection d'environ 2 ppm en poids) en sortie du réacteur tubulaire contenant la résine.

### EXEMPLE 5

Soit un brut de DMDS contenant 239 ppm de CH₃SSH (ces 239 ppm peuvent redonner dans le temps 140 ppm de CH₃SH dans le produit fini).

Ce DMDS est envoyé sur un lit de résine basique Amberlyst® A21 de Rohm & Haas préalablement séchée (5 g, soit 14,3 mL) à raison de 2000 g/h (1904 mL/h soit une VVH de 133 h⁻¹) pendant 2 heures et à une température de 20°C.

Le CH₃SSH n'est plus détectable par chromatographie gazeuse (limite de détection : environ 2 ppm en poids) en sortie du réacteur tubulaire contenant la résine.

Le DMDS utilisé dans cet exemple qui contenait initialement 441 ppm de trisulfure de diméthyle (DMTS), en contient après passage sur la résine 809 ppm, soit une augmentation de 368 ppm. Cette valeur de 368 ppm est très proche du maximum théorique qui est de 376 ppm (valeur que l'on obtient en considérant que chaque mole de CH₃SSH a donné une mole de DMTS).

## Revendications

1. Procédé de préparation de disulfure de diméthyle, comprenant au moins les étapes suivantes :
a) oxydation par le soufre de méthylmercaptan, par catalyse basique, pour obtenir un premier brut réactionnel contenant majoritairement le disulfure de diméthyle ;
b) stripping du H₂S formé ;
c) distillation du méthylmercaptan résiduel ;
d) séparation des produits lourds (polysulfures) ;
e) étêtage final permettant d'éliminer les dernières traces des composés volatils ;
f) mise en contact du flux en sortie de l'étape d), avec un catalyseur basique permettant l'élimination de l'hydrodisulfure de méthyle par conversion dudit hydrodisulfure de méthyle en sulfure ou polysulfures de diméthyle ;
g) récupération du disulfure de diméthyle à teneur en hydrodisulfure de méthyle inférieure à 50 ppm en poids.

2. Procédé selon la revendication 1, dans lequel l'étape f) d'élimination de l'hydrodisulfure de méthyle, en sulfure ou polysulfures de diméthyle en présence d'un catalyseur basique, est effectuée après l'étape d).

3. Procédé selon l'une quelconque des revendications précédentes, dans lequel le catalyseur basique est un catalyseur hétérogène par rapport au milieu réactionnel.

4. Procédé selon l'une quelconque des revendications précédentes, dans lequel le catalyseur basique est choisi parmi les résines échangeuses d'anions, les catalyseurs basiques sous forme amine libre, les alumines dopée à l'oxyde de sodium et/ou à l'oxyde de potassium, l'oxyde de magnésium (MgO), et autres, de préférence le catalyseur basique est une résine échangeuse d'anions.

## Patentansprüche

1. Verfahren zur Herstellung von Dimethyldisulfid, das mindestens die folgenden Schritte umfasst:
a) Oxidieren von Methylmercaptan mit Schwefel durch Basenkatalyse zum Erhalt eines ersten Reaktionsrohprodukts, das hauptsächlich das entsprechende Dimethyldisulfid enthält;
b) Abstrippen des gebildeten H₂S;
c) Abdestillieren des restlichen Methylmercaptans;
d) Abtrennen der schweren Produkte (Polysulfide);
e) abschließendes Toppen, was die Entfernung der letzten Spuren flüchtiger Verbindungen erlaubt;
f) Inkontaktbringen des Stroms aus Schritt d) mit einem basischen Katalysator, der die Entfernung des Methylhydrodisulfids durch Umwandlung des Methylhydrodisulfids in Dimethylsulfid oder Dimethylpolysulfide erlaubt;
g) Gewinnen des Dimethyldisulfids mit einem Gehalt an Methylhydrodisulfid von weniger als 50 Gew.-ppm.

2. Verfahren nach Anspruch 1, bei dem der Schritt f) zur Entfernung des Methylhydrodisulfids in Dimethylsulfid oder Dimethylpolysulfide in Gegenwart eines basischen Katalysators nach Schritt d) durchgeführt wird.

3. Verfahren nach einem der vorhergehenden Ansprüche, bei dem es sich bei dem basischen Katalysator um einen bezüglich des Reaktionsmediums heterogenen Katalysator handelt.

4. Verfahren nach einem der vorhergehenden Ansprüche, bei dem der basische Katalysator aus Anionenaustauscherharzen, basischen Katalysatoren in freier Aminform, mit Natriumoxid und/oder Kaliumoxid dotierten Aluminiumoxiden, Magnesiumoxid (MgO) und dergleichen ausgewählt wird und es sich vorzugsweise bei dem basischen Katalysator um ein Anionenaustauscherharz handelt.

## Claims

1. Process for preparing dimethyl disulphide, comprising at least the following steps:
a) oxidation of methyl mercaptan with sulphur, by basic catalysis, so as to obtain a first reaction crude containing predominantly the corresponding dimethyl disulphide;
b) stripping of the H2S formed;
c) distillation of the residual methyl mercaptan;
d) separation of the heavy products (polysulphides);
e) final topping making it possible to eliminate the last traces of volatile compounds;
f) bringing the stream exiting step d) into contact with a basic catalyst enabling the elimination of the methyl hydrodisulphide by conversion of said methyl hydrodisulphide to dimethyl sulphide or dimethyl polysulphides;
g) recovery of the dimethyl disulphide containing a methyl hydrodisulphide content of less than 50 ppm by weight.

2. Process according to Claim 1, in which step f) for eliminating the methyl hydrodisulphide into dimethyl sulphide or dimethyl polysulphides, in the presence of a basic catalyst, is performed after step d).

3. Process according to either one of the preceding claims, in which the basic catalyst is a heterogeneous catalyst with respect to the reaction medium.

4. Process according to any one of the preceding claims, in which the basic catalyst is chosen from anion exchange resins, basic catalysts in free amine form, aluminas doped with sodium oxide and/or with potassium oxide, magnesium oxide (MgO), and the like, preferably the basic catalyst is an anion exchange resin.
